# FASCICULE DE BREVET EUROPEEN

(11) **EP 0 410 061 B1**
(45) Date de publication et mention de la délivrance du brevet: **27.04.1994**
(21) Numéro de dépôt: 89402513.9
(22) Date de dépôt: 14.09.1989
(51) Int. Cl.: A23J 3/00, C12P 21/06

(54) **Procédé de traitement de solutions protéiques contenant des pigments tels que groupements héminiques ou chlorophylles en vue de leur décoloration et produits obtenus**
Verfahren zur Behandlung und Entfärbung von Proteinlösungen, die Häm- und Chlorophyll-Pigmente enthalten, und entsprechende Produkte
Process for the treatment and decolorisation of protein solutions containing pigments from the groups haemo and chlorophyll, and products obtained

(30) Priorité: 27.07.1989 EP 89402138
(43) Date de publication de la demande: 30.01.1991
(73) Titulaire: CIBEVIAL, F-69960 Corbas (FR); IMEDEX, F-69007 Lyon (FR)
(72) Inventeur: Coves, Jacques, F-38000 Grenoble (FR); Tayot, Jean-Louis, F-69890 La Tour de Salvagny (FR)
(74) Mandataire: Bernasconi, Jean

(56) Documents cités:
- FR-A- 2 340 691
- FR-A- 2 415 968

## Description

Les déposantes ont déjà décrit, dans la demande de brevet français FR-A-8810405 et la demande de brevet européen EP-A-0 354 100, publiées postérieurement à la date de dépôt de la présente demande, un procédé de traitement de solutions protéiques contenant des pigments tels que groupements héminiques ou chlorophylles en vue de leur décoloration et les produits obtenus par ce procédé.

Le procédé décrit dans les demandes précitées consiste à soumettre, dans une première étape, la solution protéique à une hydrolyse enzymatique légère effectuée à un pH et à une température adaptée à l'activité de l'enzyme utilisé, puis, dans une seconde étape, à porter la solution partiellement hydrolysée à une température supérieure à 60°C, à un pH acide, notamment compris entre 2 et 4 ou 5, permettant de fluidifier la solution obtenue, et de provoquer l'agrégation des groupements colorés.

Ce procédé remédie aux inconvénients des procédés de décoloration de l'hémoglobine de l'art antérieur parmi lesquels on peut citer:
- TYBOR et Coll. - Journal of Food Science 38, 4-6, (1973)
- Demande de brevet PCT WO81/02834 (LINDROOS)
- Demande de brevet japonais JP-A-55-008261 (SATO et HAYAKAWA)
- AUTIO et Coll. - Journal of Food Science (1980), 49, 859-862
- Demande de brevet PCT/FR82/00184 (ESPENAN)
- REGNIER, Revue Technique des Vétérinaires et de l'Alimentation (R.T.V.A.), 22, 23-25, (1983)
- Demande de brevet FR-A-2 415 968
- Demande de brevet européen EP-A-0159231.

Pour la discussion de ces documents, il est renvoyé à la demande de brevet européen précitée des déposantes.

La demande de brevet FR-A-2415968 décrit un procédé de décoloration des substances issues du sang animal, consistant à hémolyser la fraction de globules rouges du sang par addition d'eau, à soumettre l'hémolysat à une hydrolyse partielle enzymatique, et à une élévation de température, le surnageant obtenu étant seulement partiellement décoloré, ce surnageant étant ensuite décoloré par le charbon actif.

D'après la demande de brevet français FR-A-2 340 691, on connaît également un procédé d'hydrolyse d'un concentré d'hématies d'un sang animal, l'hydrolyse pouvant être effectuée soit par voie enzymatique à l'aide d'enzymes pancréatiques ou de la papaïne, puis en amenant le pH à une valeur comprise entre 6 et 8 en utilisant un acide minéral ou carboxylique, soit directement à l'aide d'acide chlorhydrique ou d'acide sulfurique en lieu et place de l'hydrolyse enzymatique. L'acide sulfurique n'est jamais indiqué en liaison avec le traitement par voie enzymatique.

Au titre de l'arrière-plan technologique, on peut citer également :
- FSTA, 84-09-s1944, 84057456; F. JACOBSEN et al.: "Decoloration of slaughterhouse blood by an enzyme process" International Union of Food Science and Technology 6th Congress, Enzymes R & D, Novo industri A/S, DK-2880 Bagswaerd, Denmark, & Proceeding of the 6th International Congress of Food Science and technology 9 ref. 2, 179-180, 1983.
- Brevet US-A-4.250.197
Le procédé décrit dans les demandes de brevet précitées des déposantes permet, par rapport aux procédés de l'état de la technique, de fournir un procédé de décoloration de solutions protéiques contenant des groupements pigmentés, qui n'utilise ni solvants organiques ni adsorbants, tout en permettant de récupérer les protéines avec des rendements très élevés et en obtenant une excellente décoloration. Il est ainsi possible de valoriser les substances protéiques pigmentées, y compris le sang et plus particulièrement l'hémoglobine, notamment le sang d'abattoir et plus particulièrement la fraction lourde appelée cruor qui contient principalement les hématies après séparation du plasma.

Ce procédé est particulièrement remarquable par le fait qu'après hydrolyse enzymatique légère, la solution partiellement hydrolysée est portée à une température supérieure à 60°C, à un pH acide, notamment compris entre 2 et 4 ou 5, le moyen utilisé pour ajuster le pH à sa valeur étant l'acide sulfurique, lequel permet une mise en oeuvre optimale du procédé.

D'une façon générale, le choix de l'enzyme utilisé, parmi les enzymes protéolytiques du commerce dont le prix est compatible avec une exploitation industrielle d'un procédé ayant, par exemple, des applications dans le domaine alimentaire, n'est pas critique et le pH approprié (de même que la température) peut facilement être celui auquel l'enzyme en question est habituellement actif.

La présente invention repose sur la constatation surprenante que l'un de ces enzymes courants, à savoir la papaïne, donne des résultats particulièrement intéressants pour les hémoglobines issues d'espèces animales autres que le boeuf et, bien entendu, également pour le cruor bovin lui-même.

L'invention a donc pour objet un procédé de traitement de solutions protéiques contenant des groupements héminiques ou chlorophylles en vue de leur décoloration, dans lequel on soumet, dans une première étape, la solution protéique à une hydrolyse enzymatique légère par la papaïne, à un pH, habituel pour la papaïne, de 5 à 8 et à une température, habituelle pour la papaïne, de 65 à 75° C, puis, dans une deuxième étape, on porte la solution partiellement hydrolysée à une température supérieure à 60° C, notamment de l'ordre de 70° C, et à un pH de valeur inférieure ou égale à 5, de manière à fluidifier la solution obtenue et provoquer l'agrégation des groupements colorés, le pH étant ajusté, pour la deuxième étape, à l'aide d'acide su lfurique.

Selon l'invention, la solution protéique traitée peut être notamment du cruor bovin, du cruor de cheval, du sang de mouton.

Il est ainsi possible d'obtenir facilement un jus décoloré par simple centrifugation. De plus, les faibles variations de pH permettent de limiter au maximum la génération de sels qui doivent ensuite être éliminés.

Il va être donné ci-après, à titre non limitatif, une série d'exemples de mise en oeuvre du procédé sur cruor ou sur sang entier.

### EXEMPLE 1.

100 l de cruor bovin, contenant 270 g d'hémoglobine par litre, sont dilués par 300 l d'eau permutée, de manière à obtenir une solution à 67,5 g/l dont le pH est de 7,2.

400 g de papaïne vendue sous la dénomination T400 purifiés (pouvoir protéolytique selon CODEX 1949 > 400) en solution dans 2 l d'eau sont ajoutés et le mélange est homogénéisé par agitation.

La température est alors portée à 72°C et maintenue à cette valeur pendant 1 heure. Passé ce temps, le pH s'est stabilisé à 6,4 et 18 l d'H₂SO₄ 1 N sont introduite pour l'ajuster à 5. L'incubation est poursuivie pendant encore 30 minutes à environ 70°C.

La clarification du mélange est réalisée par filtration sur filtre rotatif.

A l'issue de cette étape, 430 l de solution décolorée sont obtenus. La concentration en protéines est de 47 g/l, ce qui correspond à un rendement de 75 %.

### EXEMPLE 2.

300 ml de cruor bovin sont ajoutés à 900 ml d'eau permutée. La solution obtenue contient 55 g de protéines par litre.

Après introduction de 1,2 g de papaïne T400 purifiée en solution dans 1 ml d'eau, la température est portée à 70°C pendant une heure.

Le pH est ensuite ajusté à 5 par 56 ml d'H₂SO₄ 1 N et la réaction se poursuit pendant 30 minutes.

Par centrifugation, on obtient environ 1 l de surnageant décoloré dont la neutralisation à pH 6,5 nécessite l'addition de 21 ml de NaOH 1N.

L'analyse de cette solution permet d'obtenir les résultats suivants :

| | |
|---|---|
| - azote total | 6,96 g/l |
| - protéines (x 6,25) | 43,5 g/l |
| - matière sèche | 46,2 g/l |
| - matières minérales | 3,7 g/l |

### EXEMPLE 3.

100 ml de cruor de cheval représentant 25 g d'hémoglobine sont mélangés à 300 ml d'eau permutée.

L'hydrolyse est réalisée à 70°C pendant 1 heure en présence de 0,4 g de papaïne T400 purifiée. La réaction se poursuit pendant 30 minutes après ajustage du pH à 5 par quelques gouttes d'H₂SO₄ concentré.

Ensuite, 320 ml de surnageant décoloré sont récupérés par centifugation. La concentration en protéines est de 62,8 g/l; le rendement est dont supérieur à 80 %.

### EXEMPLE 4.

520 ml de sang entier de mouton contenant 169 g de protéines par litre sont ajoutés à 1 litre d'eau permutée.

L'hydrolyse est réalisée pendant 1 heure à 70°C en présence de 4 g de papaïne T400 purifiée.

Le pH est ensuite ajusté à 5 par 68 ml d'H₂SO₄ 1 N et la réaction se poursuit 30 minutes à 70°C.

Par centrifugation, 1,4 l de surnageant décoloré sont récoltés. La neutralisation de cette solution à pH 6,5 nécessite 35 ml de NaOH 1 N.

L'analyse de cette solution donne les résultats suivants:

| | |
|---|---|
| - azote total | 7,1 g/l |
| - protéines (x 6,25) | 44,4 g/l |
| - matière sèche | 50,7 g/l |
| - matières minérales | 6 g/l. |

## Revendications

1. Procédé de traitement de solutions protéiques contenant des groupements héminiques ou chlorophylles en vue de leur décoloration, dans lequel on soumet, dans une première étape, la solution protéique à une hydrolyse enzymatique légère par la papaïne, à un pH de 5 à 8 et à une température de 65 à 75° C, puis, dans une deuxième étape, on porte la solution partiellement hydrolysée à une température supérieure à 60° C et à un pH de valeur inférieure ou égale à 5 de manière à fluidifier la solution obtenue et provoquer l'agrégation des groupements colorés, le pH étant ajusté, pour la deuxième étape, à l'aide d'acide sulfurique.

2. Procédé selon la revendication 1, caractérisé en ce que la seconde étape est effectuée à une température de l'ordre de 70° C.

3. Procédé selon la revendication 1 ou 2, caractérisé en ce que la solution traitée est du cruor bovin.

4. Procédé selon la revendication 1 ou 2, caractérisé en ce que la solution traitée est du cruor de cheval.

5. Procédé selon la revendication 1 ou 2, caractérisé en ce que la solution traitée est du sang de mouton.

## Claims

1. A process for treating proteinic solutions containing heminic groups or chlorophylls in view of their decolorisation, wherein the proteinic solution is subjected, in a first step, to a slight enzymatic hydrolysis with papain, at a pH from 5 to 8 and at a temperature from 65 to 75°C, then, in a second step, the partially hydrolysed solution is brought at a temperature higher than 60°C and at a pH value lower than or equal to 5 so as to fluidify the obtained solution and to trigger the agglutination of the colored groups, the pH being adjusted, for the second step, with sulfuric acid.

2. A process according to claim 1, characterized in that the second step is carried out at a temperature of about 70°C.

3. A process according to claim 1 or 2, characterized in that the treated solution is bovine cruor.

4. A process according to claim 1 or 2, characterized in that the treated solution is horse cruor.

5. A process according to claim 1 or 2, characterized in that the treated solution is sheep blood.

## Patentansprüche

1. Verfahren zur Behandlung von Proteinlösungen, die Hämin- oder Chlorophyllgruppen enthalten, im Hinblick auf deren Entfärbung, in dem man in einem ersten Schritt bei einem pH von 5 bis 8 und bei einer Temperatur von 65 bis 75°C die Proteinlösung einer leichten enzymatischen Hydrolyse durch Papain unterzieht, dann in einem zweiten Schritt die teilweise hydrolysierte Lösung auf eine Temperatur oberhalb von 60°C und auf einen pH-Wert unterhalb von oder gleich 5 bringt, um die erhaltene Lösung flüssiger zu machen und die Aggregation der gefärbten Gruppen herbeizuführen, wobei dar pH beim zweiten Schritt mit Schwefelsäure eingestellt wird.

2. Verfahren nach Anspruch 1, dadurch gekennzeichnet, daß der zweite Schritt bei einer Temperatur in der Größenordnung von 70°C durchgeführt wird.

3. Verfahren nach Anspruch 1 oder 2, dadurch gekennzeichnet, daß die behandelte Lösung Rinder-Cruor ist.

4. Verfahren nach Anspruch 1 oder 2, dadurch gekennzeichnet, daß die behandelte Lösung Pferde-Cruor ist.

5. Verfahren nach Anspruch 1 oder 2, dadurch gekennzeichnet, daß die behandelte Lösung Schafsblut ist.
